# EUROPÄISCHE PATENTSCHRIFT

(11) **EP 2 638 427 B1**
(45) Veröffentlichungstag und Bekanntmachung des Hinweises auf die Patenterteilung: **04.12.2019**
(21) Anmeldenummer: 11776317.7
(22) Anmeldetag: 17.10.2011
(51) Int. Cl.: G01J 1/04, G01J 1/42, G01J 1/44, A61F 9/06, G02F 1/133

(54) **BLENDSCHUTZVORRICHTUNG**
ANTIGLARE DEVICE
DISPOSITIF ANTI-ÉBLOUISSEMENT

(30) Priorität: 15.10.2010 CH 16902010
(43) Veröffentlichungstag der Anmeldung: 18.09.2013
(73) Patentinhaber: Castelberg, Donata, 8820 Wädenswil (CH)
(72) Erfinder: GUNZ, Stefan, 8804 Au (CH); CASTELBERG, Manfred, 8824 Schönenberg (CH)
(74) Vertreter: Alder, Hans Rudi
(86) Internationale Anmeldenummer: PCT/CH2011/000244
(87) Internationale Veröffentlichungsnummer: WO 2012/048436

(56) Entgegenhaltungen:
- JP-A- 3 054 521
- US-A- 4 491 390
- US-A- 5 117 099
- US-A- 5 376 783

## Beschreibung

Die vorliegende Erfindung betrifft eine elektrooptische Blendschutzvorrichtung für Schutzbrillen, Schutzhelme oder Schutzmasken gemäss Oberbegriff des Anspruch 1, sowie ein Verfahren zum Betrieb einer solchen elektrooptischen Blendschutzvorrichtung.

Derartige Blendschutzvorrichtungen sind beispielsweise aus der EP-550'384 oder WO-03/106097 hinlänglich bekannt und finden ihre Verwendung vorzugsweise in Schweissermasken. Diese Blendschutzvorrichtungen umfassen im Wesentlichen eine optische Filteranordnung mit mindestens einer Flüssigkristallzelle, deren optische Transmission mit Hilfe einer elektronischen Schaltung gesteuert wird, welche das Signal eines Sensors in gewünschter Weise verwendet. Leider erweisen sich diese Blendschutzvorrichtungen als nicht generell verwendbar und erfordern eine aufwendige Elektronik für deren spezifische Anwendung.

Störende Arbeitsplatzbeleuchtungen, wie sie von Lichtröhren erzeugt werden, werden mit mässigem Erfolg durch sogenannte "Daylight-Filter" unterdrückt. Diese "Daylight-Filter" sind auf den Sensoren angebracht und absorbieren flackerndes Licht mit Wellenlängen von kleiner als ca. 700 nm.

Es ist deshalb mit der EP-0'579'076, resp. US-5,880,793 auch schon vorgeschlagen worden optische und nichtoptische Sensoren in geeigneter Kombination logisch und/oder zeitlich miteinander zu verknüpfen, um Störquellen zu erkennen, d.h. die Störsignale von den Nutzsignalen zu trennen und gezielt aus den Steuersignalen für die Flüssigkristallzellen der verwendeten Blendschutzscheiben herauszufiltern. All diese bekannten Blendschutzvorrichtungen basieren darauf ein Flackern zu detektieren, d.h. rasche Helligkeitsänderungen von wenigen kHz, meist im infraroten Bereich, auszumachen. Die dazu verwendeten optischen Sensoren detektieren immer nur die Beleuchtungsstärke (Lux) eines Objektfeldes.

Die Bestrebungen der heutigen Schweissmaschinenhersteller zielen jedoch auf die Erzeugung von ruhigen und möglichst kleinen Schweissflammen. Dafür sind die herkömmlichen Filteranordnungen nicht besonders geeignet und wird die Erkennbarkeit der Helligkeitsänderungen immer schlechter. Bei bekannten Vorrichtungen wird deshalb die Empfindlichkeit der Sensoren gesteigert, was nicht nur den elektronischen Aufwand und erforderlichen Stromkonsum erhöht, sondern auch zu ungewolltem Verdunkeln der Blendschutzscheibe führt.

Eine bevorzugte Verwendung derartiger Blendschutzvorrichtungen in Schutzbrillen ist aus der WO-95/22074 ersichtlich. Der mechanische Aufbau solcher Schutzbrillen ist komplex und führt zu unerwünscht schweren Produkten. Insbesondere ist die Stromführung über mehrere voneinander separierbare Bauteile störanfällig auf mechanische und chemische Belastungen (Schläge, Feuchtigkeit, etc.), wie sie auf dem Gebiet der Schweissertechnik bekannt
US4491390 offenbart die Merkmale der Präambel des Anspruchs 1.

Es ist deshalb die Aufgabe der vorliegenden Erfindung die Nachteile der bekannten Blendschutzvorrichtungen zu überwinden und insbesondere eine Blendschutzvorrichtung und ein Verfahren zum Betrieb einer solchen zu schaffen, welche ohne aufwändige Elektronik auch bei ruhigem und wenig intensivem Flammenlicht und für unterschiedlichste Anwendungen auch bei extremen Bedingungen langfristig und sicher verwendbar ist.

Diese Aufgabe wird erfindungsgemäss mit einer Blendschutzvorrichtung mit den Merkmalen des Anspruch 1 gelöst und insbesondere mit einer elektrooptische Blendschutzvorrichtung mit mindestens zwei optischen Sensoren zur Detektion der Leuchtdichte [cd/m²] eines Objektfeldes, welche Sensoren eine unterschiedliche Charakteristik für deren winkelabhängige relative Empfindlichkeit (S₀) - im Folgenden auch Detektionskeule genannt - aufweisen.

In einer bevorzugten Ausführungsform der vorliegenden Erfindung umfassen die optischen Sensoren mindestens eine erste Fotodiode mit einem schmalen Detektionswinkel θ₁ < 45° und eine zweite Fotodiode mit einem breiten Detektionswinkel θ₂ > 45°. Vorzugsweise sind dazu die Fotodioden mit optisch unterschiedlich stark brechenden Linsen versehen.

Die elektronische Schaltung zur Steuerung der Transmission der Flüssigkristallzelle weist eine Differenzschaltung auf, welche Differenzschaltung aus den von den jeweiligen Sensoren erzeugten Fotoströmen einen Differenzstrom, resp. ein Differenzsignal bildet, welches als Steuersignal einem Komparator mit interner oder externer Referenz zugeleitet wird.

Es versteht sich, dass die elektronische Schaltung zur Steuerung der Transmission der Flüssigkristallzelle zwischen der Differenzschaltung und dem Komparator einen Spannungsteiler aufweisen kann.

In einer erfindungsgemässen Ausführungsform der vorliegenden Blendschutzvorrichtung ist der Komparator permanent an der Stromversorgung angeschlossen und generiert nur bei einem vorgegebenen Schwellwert des Differenzsignals ein Eingangssignal für die Flüssigkristallzellen-Treiberschaltung. Damit ist die elektronische Schaltung zur Steuerung der optischen Transmission der Flüssigkristallzellen automatisch ein- und ausschaltbar und lässt sich die gesamte elektronischen Schaltung zur Steuerung der optischen Transmission bspw. in die Fassung der mindestens einen Flüssigkristallzelle eingiessen.

Das erfindungsgemässe Verfahren zum Betrieb einer solchen elektrooptischen Blendschutzvorrichtung zeichnet sich dadurch aus, dass mit Hilfe einer elektronischen Differenzschaltung aus den von mindestens zwei optischen Sensoren - mit unterschiedlicher Charakteristik für die winkelabhängige relative Empfindlichkeit - erzeugten Fotoströmen ein Differenzstrom gebildet wird, welcher die optische Transmission einer Flüssigkristallzelle steuert. Der so erzeugte Differenzstrom wird einem Komparator mit interner oder externer Referenz zugeführt, dessen Signal die optische Transmission der Flüssigkristallzelle steuert.

Die erfindungsgemässe Blendschutzvorrichtung lässt sich universell verwenden und ist für Sonnenbrillen, für Arbeitsschutzbrillen - wie sie bspw. Goldschmiede beim Hartlöten verwenden - oder für Schweisserschutzmasken - wie sie beim Schweissen in der Metallindustrie verwendet werden - geeignet.

Insbesondere lässt sich die gesamte Elektronik, also inkl. Stromversorgung, in die Halterung eingiessen und ist die Blendschutzvorrichtung damit vor jedwelchen mechanischen und chemischen Beschädigungen geschützt. Die Funktionsfähigkeit ist über lange Zeit gewährleistet, da die Treiberschaltung für die mindestens eine Flüssigkristallzelle nur bei vorliegen eines vorgegebenen Schwertsignals aktiviert wird.

Im Folgenden soll anhand der Figuren ein Ausführungsbeispiel näher erläutert werden. Dabei zeigt:
- Fig. 1:: ein Schaltbild für die erfindungsgemässe Erzeugung eines Differenzstoms zur Steuerung einer Flüssigkristallzelle;
- Fig. 2:: ein Diagramm zur Winkelabhängigkeit der relativen Empfindlichkeit der Sensoren;
- Fig. 3:: ein Diagramm zur Winkelabhängigkeit der Fotoströme der Sensoren;
- Fig. 4:: ein Kombi-Schaltbild zu bevorzugten Ausführungsformen der erfindungsgemässen Blendschutzvorrichtung.

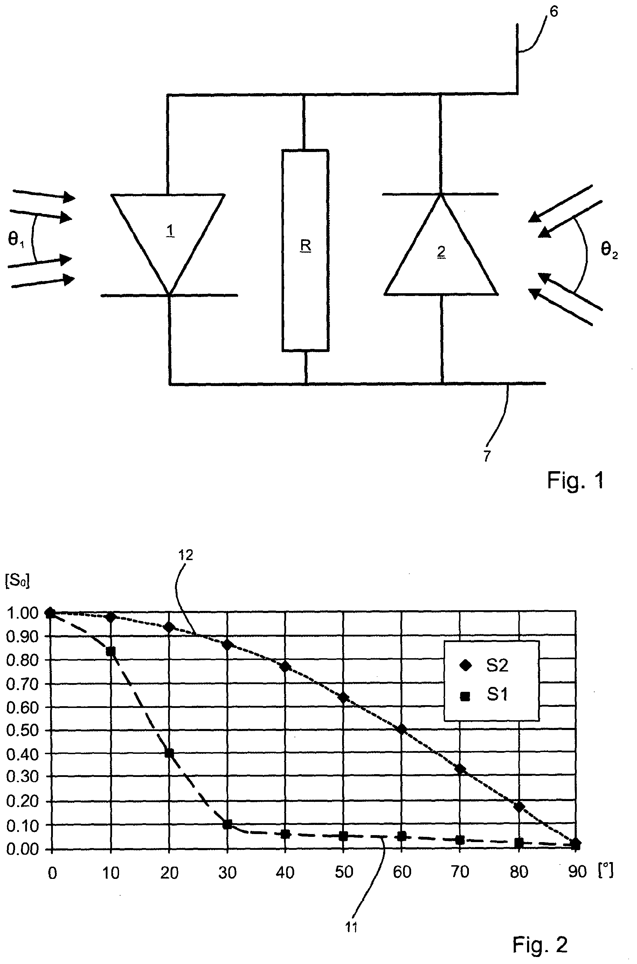

Das in Fig. 1 dargestellte Schaltbild zeigt eine erste einfache Ausführungsform für die elektronische Verknüpfung der optischen Sensoren. Ein erster Sensor mit einer ersten Fotodiode 1 weist einen schmalen Detektionswinkel θ₁ auf, während ein zweiter Sensor mit einer zweiten Fotodiode 2 einen breiten Detektionswinkel θ₂ aufweist. Für eine bevorzugte Anwendung beträgt der schmale Detektionswinkel θ₁ ca. 30°, während der breite Detektionswinkel θ₂ ca. 120° aufweist. Diese unterschiedlichen Detektionswinkel können vom Fachmann durch geeignete Wahl handelsüblicher Fotodioden und/oder durch das Anbringen von geeigneten optischen Linsen an diesen Fotodioden in gewünschter Weise an die vorgesehene Anwendung angepasst werden. Mit der Schaffung unterschiedlich weiter Detektionskegel oder -keulen wird die Leuchtdichte [cd/m²] für unterschiedliche Detektionsfelder detektiert. Durch die parallel und gegenläufig geschaltete Anordnung der Fotodioden 1 und 2 werden deren Fotoströme i₁, i₂ subtraktiv überlagert. Die so erzeugten Signale werden über Leiter 6, 7 einem Komparator (IC1) zugeführt, welcher ein Steuersignal für die Steuerung der optischen Transmission der Flüssigkristallzelle (LCD) generiert. Es versteht sich, dass dieser Komparator dazu mit einer externen oder internen Referenz gekoppelt ist.

Eine typische Charakteristik für die winkelabhängige relative Empfindlichkeit (S₀) der erfindungsgemäss verwendeten Fotodioden ist aus dem Diagramm in Fig. 2 ersichtlich. Dabei zeigt die Kurve 11 den winkelabhängigen Verlauf eines ersten Sensors S₁ mit schmalem Detektionswinkel (θ₁ < 45°). Diese lässt eine hohe relative Empfindlichkeit S₀ im Halbwinkelbereich von 0° bis 15° erkennen, resp. zeigt keine relevante relative Empfindlichkeit S₀ im Halbwinkelbereich oberhalb 30°. Demgegenüber weist der Verlauf der Kurve 12 für die relative Empfindlichkeit S₀ eines zweiten Sensors S₂ mit breitem Detektionswinkel (θ₂ > 45°) im Halbwinkelbereich von 0° bis 15° sehr hohe Werte auf, welche sich gegen den Halbwinkelbereich von 90° kontinuierlich verringern.

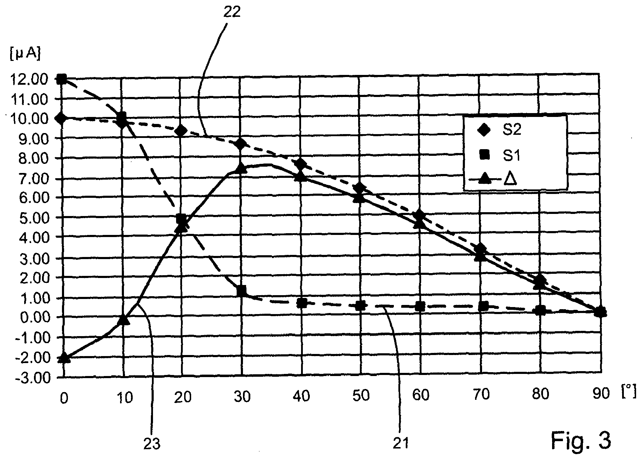

Das in Fig. 3 gezeigte Diagramm macht die Winkelabhängigkeit der jeweiligen Fotoströme i₁, i₂ der Sensoren S₁, S₂ deutlich. Dabei entspricht der Verlauf der Kurve 21 für die Stromstärke [µA] des Fotostroms i₁ im Wesentlichen dem Verlauf der Kurve 11 aus Fig. 1 für die winkelabhängige relative Empfindlichkeit S₀ des ersten Sensors S₁ mit schmalem Detektionswinkel θ₁. Der Verlauf der Kurve 22 für die Stromstärke [µA] des Fotostroms i₂ entspricht im Wesentlichen dem Verlauf der Kurve 12 aus Fig. 2 für die winkelabhängige relative Empfindlichkeit S₀ des zweiten Sensors S₂ mit breitem Detektionswinkel θ₂. Kurve 23 zeigt die subtraktive Überlagerung dieser beiden Charakteristiken und macht deutlich, dass durch die Detektion der unterschiedlichen Leuchtdichte [Lichtstärke pro Fläche, cd/m²] anstelle der Beleuchtungsstärke [Lux] ein aussergewöhnlich zweckmässiges Steuersignal in überraschend einfacher Weise erzeugt werden kann.

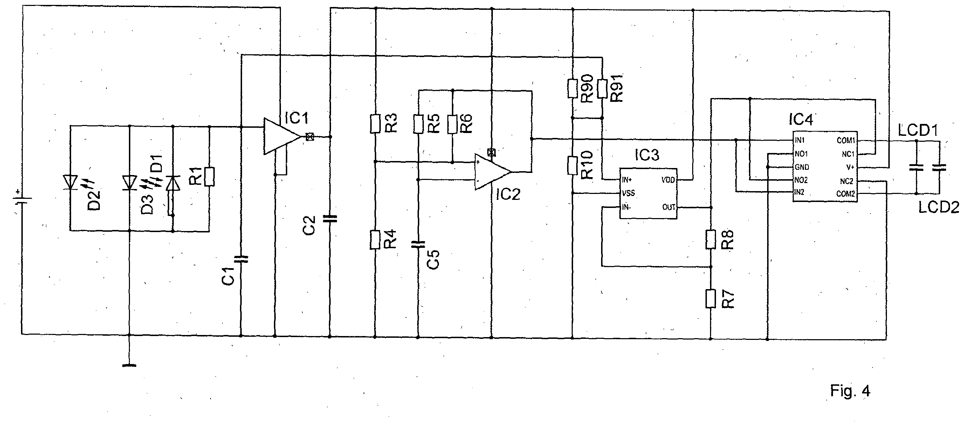

Bevorzugte Ausführungsformen der erfindungsgemässen Vorrichtung sind in Fig. 4 dargestellt und macht die Funktionsweise deutlich. Für die Stromversorgung ist eine handelsübliche Knopfzelle, hier eine Lithiumzelle mit 30mAh Kapazität, vorgesehen. Es versteht sich, dass auch andere Spannungsquellen, insbesondere Solarzellen, verwendet werden können, welche in der Regel mehr Platz benötigen und damit das ursprüngliche Design der Schutzbrillen, Schutzhelme oder Schutzmasken stark beeinflussen. Durch die Verwendung einer geeigneten internen oder externen Referenz (Leuchtdichte-Schwelle) dient der Komparator IC1 als Schwellwertschalter und generiert bei vorgegebener Leuchtdichtedifferenz in einer ersten Variante dieser Ausführungsform ein von einem zweiten Komparator IC2 weiter aufbereitetes Steuersignal für die LCD-Treiberschaltung IC4. Damit schaltet die mindestens eine Flüssigkristallzelle LCD1, LCD2 bei Überschreiten einer durch interne oder externe Spannungsteiler vorgegebenen Komparatorschwelle auf einen festen, der Verwendung der Flüssigkristallzelle angepassten Transmissionswert. Der durch die Verwendung dieser Komparatoren IC1, IC2 minimierte Stromverbrauch führt mit den zurzeit handelsüblichen Knopfzellen zu einer Lebensdauer von bis zu 7 Jahren und erlaubt es, die gesamte Elektronik, d.h. Stromversorgung, Sensoren, elektronische Schaltung und Flüssigkristallzellenkontakte, vollständig und wasserdicht zu vergiessen. In einer zweiten Variante dieser Ausführungsform wird der Differenzstrom i_{Δ} direkt einem Operationsverstärker IC3 zugeführt, welcher der LCD-Treiberschaltung IC4 als Taktgenerator dient. Damit lässt sich die optische Transmission der mindestens einen Flüssigkristallzelle LCD1, LCD2 mit Hilfe des, von der Differenzschaltung (Fig. 1) in Abhängigkeit der Leuchtdichte erzeugten und über einen Operationsverstärker IC3 geführten Spannungssignals, stufenlos regeln.

Die gewünschte Schaltung lässt sich vom Fachmann auf diesem Gebiet mit Bestückungsautomaten in der einen oder anderen Variante auf einer konventionellen Leiterplatte aufbauen, weiter miniaturisieren und in kompakter Weise in jede Fassung oder Halterung integrieren. Insbesondere sind mindestens die Stromversorgung, die zwei Sensoren und die elektronische Schaltung zur Steuerung der optischen Transmission der mindestens einen Flüssigkristallzelle in einer solchen Fassung oder Halterung für die mindestens eine Flüssigkristallzelle integriert. Dies erlaubt es, die erfindungsgemässe Blendschutzvorrichtung in feuchter, korrosionsfördernder oder anderer die Elektronik schädigender Umgebung auch langfristig zu verwenden. Insbesondere lässt sich damit eine Blendschutzvorrichtung schaffen, bei welcher der in der Fassung oder Halterung fest montierte optische Blendschutz mit integrierter Elektronik durch auswechselbare äussere Schutzgläser ergänzbar ist. Die äusseren Schutzgläser einer solchen modisch geformte Schutzbrille, Schutzhelms oder Schutzmaske sind vorteilhafterweise selbst beschichtet und nach oben verschwenkbar, um die empfindlichen Flüssigkristallzellen der Blendschutzvorrichtung freizugeben.

Andere Ausführungsformen dieser Vorrichtung und Verfahren zum Betrieb derselben, welche auf der erfindungsgemässen Detektion der Leuchtdichte eines Arbeitsplatzes basieren, liegen im gewöhnlichen Handeln des Fachmanns.

Die Funktionsweise, der Aufbau und die Vorteile der vorliegenden Erfindung sind dem Fachmann aus der vorliegenden Beschreibung und den dazugehörigen Figurenzeichnungen unmittelbar ersichtlich und sind insbesondere darin zu sehen, dass diese Vorrichtung permanent funktioniert und universell verwendbar ist, d.h. sich für die Verwendung in einer Sonnenbrille ebenso eignet, wie in Arbeitsschutzbrillen, bspw. zum Hartlöten von Schmuckstücken oder zum Autogenschweissen von Eisenbahnschienen oder Schiffsrümpfen. Die erforderliche Schutzstufe von bis zu 5 oder 6, d.h. für eine optische Transmission von ca. 1%, lässt sich mit zusätzlichen Verstärkern ebenfalls problemlos erreichen. Die genannten Schwierigkeiten in der Elektronik fallen mit der vorliegenden Erfindung weg, z.B. die Unterdrückung tiefer Flackerfrequenzen, speziell aber die relativ schnellen, stromintensiven und/oder breitbandigen Verstärker.

## Patentansprüche

1. Elektrooptische Blendschutzvorrichtung für Schutzbrillen, Schutzhelme oder Schutzmasken mit mindestens zwei Sensoren und einer elektronischen Schaltung zur Steuerung der optischen Transmission mindestens einer Flüssigkristallzelle (LCD1, LCD2), wobei die mindestens zwei Sensoren optische Sensoren (S1, S2) zur Detektion der Leuchtdichte [cd/m²] eines Objektfeldes sind, welche Sensoren (S1, S2) eine unterschiedliche Charakteristik für deren winkelabhängige relative Empfindlichkeit (S₀) aufweisen, **dadurch gekennzeichnet dass** mindestens ein erster optischer Sensor (S1) einen Detektionswinkel θ1 < 45° aufweist und mindestens ein zweiter optischer Sensor (S2) einen Detektionswinkel θ2 > 45° umfasst, wobei die winkelabhängige relative Empfindlichkeit (S₀) des mindestens ersten optischen Sensors (S1) und die winkelabhängige relative Empfindlichkeit (S₀) des mindestens zweiten optischen Sensors (S2) mindestens im Halbwinkelbereich von 0° bis 15° hohe d.h. sich überlappende Werte aufweisen.

2. Elektrooptische Blendschutzvorrichtung nach Anspruch 1, **dadurch gekennzeichnet, dass** die optischen Sensoren (S1, S2) Fotodioden (1, 2) umfassen, welche mit optisch unterschiedlich stark brechenden Linsen versehen sind.

3. Elektrooptische Blendschutzvorrichtung nach einem der Ansprüche 1 bis 2, **dadurch gekennzeichnet, dass** die elektronische Schaltung zur Steuerung der Transmission der mindestens einen Flüssigkristallzelle (LCD1, LCD2) eine Differenzschaltung aufweist, welche aus den von den jeweiligen Sensoren (S1, S2) erzeugten Fotoströmen (i₁, i₂) einen Differenzstrom (i_{Δ}) bildet.

4. Elektrooptische Blendschutzvorrichtung nach Anspruch 4, **dadurch gekennzeichnet, dass** die elektronische Schaltung zur Steuerung der Transmission der mindestens einen Flüssigkristallzelle mindestens einen den Differenzstrom (i_{Δ}) aufnehmenden Komparator (IC1, IC2) mit interner oder externer Referenz umfasst.

5. Elektrooptische Blendschutzvorrichtung nach Anspruch 4, **dadurch gekennzeichnet, dass** die elektronische Schaltung zur Steuerung der Transmission der mindestens einen Flüssigkristallzelle (LCD1, LCD2) derart ausgelegt ist, dass entweder ein fester oder ein stufenloser Transmissionswert erzeugbar ist.

6. Elektrooptische Blendschutzvorrichtung nach Anspruch 1, **dadurch gekennzeichnet, dass** die elektronischen Schaltung zur Steuerung der optischen Transmission mit Hilfe eines permanent versorgten Schwellwertschalter (IC1) ein- und ausschaltbar ist.

7. Elektrooptische Blendschutzvorrichtung nach Anspruch 1, **dadurch gekennzeichnet, dass** die elektronischen Schaltung zur Steuerung der optischen Transmission in die Fassung oder Halterung derselben integriert, d.h. eingegossen ist.

8. Verfahren zum Betrieb einer elektrooptischen Blendschutzvorrichtung gemäss Anspruch 1, **dadurch gekennzeichnet, dass** mit Hilfe einer elektronischen Differenzschaltung (Fig. 1) aus von mindestens zwei, die Leuchtdichte [cd/m²] messende Sensoren - mit unterschiedlicher Charakteristik für die winkelabhängige relative Empfindlichkeit (S₀) -, erzeugten Fotoströmen (i₁, i₂) ein Differenzstrom (i_{Δ}) gebildet wird, welcher die optische Transmission der mindestens einen Flüssigkristallzelle steuert.

9. Schutzbrille, Schutzhelm oder Schutzmaske mit einer elektrooptischen Blendschutzvorrichtung gemäss Anspruch 1, nämlich mit mindestens zwei Sensoren (S1, S2) und einer elektronischen Schaltung zur Steuerung der optischen Transmission mindestens einer Flüssigkristallzelle (LCD1, LCD2), **dadurch gekennzeichnet, dass** die mindestens zwei Sensoren optische Sensoren zur Detektion der Leuchtdichte [cd/m²] eines Objektfeldes sind, welche Sensoren eine unterschiedliche Charakteristik für deren winkelabhängige relative Empfindlichkeit (S₀) aufweisen.

## Claims

1. Electro-optical glare protection device for protective goggles, safety helmets or face guards with at least two sensors and an electronic switch for controlling the optical transmission of at least one liquid crystal cell (LCD1, LCD2), wherein the at least two sensors are optical sensors (S1, S2) for detecting the light density [cd/m²] of an object field, said sensors (S1, S2) having a different characteristic for their angle-dependent relative sensitivity (S₀), **characterised in that** at least one first optical sensor (S1) has a detection angle θ1 < 45°, and that at least one second optical sensor (S2) comprises a detection angle θ2 > 45°, wherein the angle-dependent relative sensitivity (S₀) of the at least first optical sensor (S1) and the angle-dependent relative sensitivity (S₀) of the at least second optical sensor (S2) has high, i.e. overlapping values at least in the half angle range from 0° to 15°.

2. Electro-optical glare protection device according to claim 1, **characterised in that** the optical sensors (S1, S2) comprise photodiodes (1, 2), which are equipped with optically different, highly diffractive lenses.

3. Electro-optical glare protection device according to one of the claims 1 to 2, **characterised in that** the electronic switch for controlling the transmission of the at least one liquid crystal cell (LCD1, LCD2) has a difference switch, which forms a difference current (i_{Δ}) from the photocurrents (i₁, i₂) generated by the respective sensors (S1, S2).

4. Electro-optical glare protection device according to claim 4, **characterised in that** the electronic switch for controlling the transmission of the at least one liquid crystal cell comprises at least one comparator (IC1, IC2) with an internal or external reference, which receives the difference current (i_{Δ}).

5. Electro-optical glare protection device according to claim 4, **characterised in that** the electronic switch for controlling the transmission of the at least one liquid crystal cell (LCD1, LCD2) is designed in such a way that either a fixed or a continuous transmission value can be generated.

6. Electro-optical glare protection device according to claim 1, **characterised in that** the electronic switch for controlling the transmission of the at least one liquid crystal cell can be switched on and off with the aid of a permanently supplied threshold value switch (IC1).

7. Electro-optical glare protection device according to claim 1, **characterised in that** the electronic switch for controlling the optical transmission is integrated, i.e. moulded into the frame or holder of the same.

8. Method for operating an electro-optical glare protection device according to claim 1, **characterised in that** a difference current (i_{Δ}) is formed with the aid of an electronic difference switch (Fig. 1) from at least two sensors measuring the light density [cd/m²] of the photocurrents (i₁, i₂) generated - with a different characteristic for the angle-dependent relative sensitivity (S₀)-,which controls the optical transmission of the at least one liquid crystal cell.

9. Protective goggles, safety helmet or face guard with an electro-optical glare protection device according to claim 1, namely with at least two sensors (S1, S2) and an electronic switch for controlling the optical transmission of at least one liquid crystal cell (LCD1, LCD2), **characterised in that** the at least two sensors are optical sensors for detecting the light density [cd/m²] of an object field, said sensors having a different characteristic for their angle-dependent relative sensitivity (S₀).

## Revendications

1. Dispositif anti-éblouissement électro-optique pour lunettes de protection, casques de protection ou masques de protection avec au moins deux capteurs et un circuit électronique destiné à commander la transmission optique d'au moins une cellule à cristaux liquides (LCD1, LCD2), dans lequel les au moins deux capteurs présentent des capteurs optiques (S1, S2) destinés à détecter la luminance [cd/m²] d'un champ d'objet, lesquels capteurs (S1, S2) présentent une caractéristique différente pour leur sensibilité angulaire relative (S₀), **caractérisé en ce qu'**au moins un premier capteur optique (S1) présente un angle de détection θ1 < 45° et au moins un second capteur optique (S2) comprend un angle de détection θ2 > 45°, dans lequel la sensibilité angulaire relative (S₀) de l'au moins premier capteur optique (S1) et la sensibilité angulaire relative (S₀) de l'au moins second capteur optique (S2) présentent au moins dans la plage de demi-angle de 0° à 15° des valeurs élevées, c'est-à-dire se chevauchant.

2. Dispositif anti-éblouissement électro-optique selon la revendication 1, **caractérisé en ce que** les capteurs optiques (S1, S2) comprennent des photodiodes (1, 2), lesquelles sont pourvues de lentilles optiquement plus ou moins fortement réfringentes.

3. Dispositif anti-éblouissement électro-optique selon l'une quelconque des revendications 1 à 2, **caractérisé en ce que** le circuit électronique destiné à commander la transmission de l'au moins une cellule à cristaux liquides (LCD1, LCD2) présente un circuit différentiel, lequel forme un courant différentiel (i_{Δ}) à partir des courants photo-électriques (i₁, i₂) produits par les capteurs (S1, S2) respectifs.

4. Dispositif anti-éblouissement électro-optique selon la revendication 4, **caractérisé en ce que** le circuit électronique destiné à commander la transmission de l'au moins une cellule à cristaux liquides comprend au moins un comparateur (IC1, IC2), recevant le courant différentiel (i_{Δ}), à référence interne ou externe.

5. Dispositif anti-éblouissement électro-optique selon la revendication 4, **caractérisé en ce que** le circuit électronique destiné à commander la transmission de l'au moins une cellule à cristaux liquides (LCD1, LCD2) est configuré de telle sorte qu'une valeur de transmission soit fixe soit variable peut être produite.

6. Dispositif anti-éblouissement électro-optique selon la revendication 1, **caractérisé en ce que** le circuit électronique destiné à commander la transmission optique peut être activé et désactivé à l'aide d'un commutateur de valeur seuil (IC1) alimenté en permanence.

7. Dispositif anti-éblouissement électro-optique selon la revendication 1, **caractérisé en ce que** le circuit électronique destiné à commander la transmission optique est intégré, c'est-à-dire coulé, dans le support ou l'élément de retenue.

8. Procédé pour le fonctionnement d'un dispositif anti-éblouissement électro-optique selon la revendication 1, **caractérisé en ce qu'**un courant différentiel (i_{Δ}), lequel commande la transmission optique de l'au moins une cellule à cristaux liquides, est formé à l'aide d'un circuit différentiel électronique (figure 1) produit à partir d'au moins deux courants photo-électriques (i₁, i₂) produits par les capteurs mesurant la luminance [cd/m²] - avec une caractéristique différente pour la sensibilité angulaire relative (S₀).

9. Lunettes de protection, casque de protection ou masque de protection avec un dispositif anti-éblouissement électro-optique selon la revendication 1, à savoir avec au moins deux capteurs (S1, S2) et un circuit électronique destiné à commander la transmission optique d'au moins une cellule à cristaux liquides (LCD1, LCD2), **caractérisés en ce que** les au moins deux capteurs sont des capteurs optiques destinés à détecter la luminance [cd/m²] d'un champ d'objet, lesquels capteurs présentent une caractéristique différente pour leur sensibilité angulaire relative (S₀).
